# EUROPÄISCHE PATENTSCHRIFT

(11) **EP 0 488 288 B1**
(45) Veröffentlichungstag und Bekanntmachung des Hinweises auf die Patenterteilung: **22.05.1996**
(21) Anmeldenummer: 91120402.2
(22) Anmeldetag: 28.11.1991
(51) Int. Cl.: A61M 39/00, A61M 1/28

(54) **Schlauchanordnung für eine Peritonealdialyse**
Tubing set for peritoneal dialysis
Jeu de tubes pour dialyse peritonéale

(30) Priorität: 30.11.1990 US 621421
(43) Veröffentlichungstag der Anmeldung: 03.06.1992
(73) Patentinhaber: Fresenius AG, D-61350 Bad Homburg (DE)
(72) Erfinder: Folden, Thomas Irvin, Alamo, California (US); Gross, Arnold, W-6699 Freisen (DE)
(74) Vertreter: Fuchs, Luderschmidt & Partner Patentanwälte

(56) Entgegenhaltungen:
- WO-A-84/03046
- FR-A- 2 327 483
- FR-A- 2 522 969
- FR-A- 2 539 034
- GB-A- 1 474 648
- US-A- 4 306 976
- US-A- 4 655 764

## Beschreibung

Die Erfindung betrifft Mittel zur besseren Durchführung einer manuellen und automatischen Peritonealdialyse. Insbesondere stützt sich die Erfindung auf ein leistungsfähiges und einfaches Mittel zur Verbindung und Trennung des Überführungsschlauches, der von der Dialyselösung zu der Peritonealhöhle des Patienten verläuft. Die Erfindung schließt eine markierte Plastikkupplung ein, die die zugewandten und die abgewandten Segmente des Überführungsschlauches miteinander verbindet, und unterbrochen werden kann entlang der Markierung zur Trennung des Schlauches.

Nach dem Stand der Technik kommen zwei gemeinsame Techniken für die Behandlung von Patienten zur Anwendung, die ein Nierenversagen erfahren haben. Die übliche Therapie ist die Blutdialyse, bei der das Blut des Patienten durch einen Filter geführt wird, durch den die Stoffwechselprodukte aus dem Blutstrom des Patienten entfernt werden.

Die zweite Technik ist die Peritonealdialyse, bei der Lösungen zyklisch in die und aus der Peritonealhöhle des Patienten geleitet werden, wobei die Ausscheidungen mit der verbrauchten Lösung entfernt werden.

Beide Techniken arbeiten nach dem Prinzip der Diffusion über semipermeable Membranen. Im Fall der Peritonealdialyse ist die Membran, die benutzt wird, das Peritoneum des Patienten. Obgleich es nichts leistungsstärkeres gibt als die Blutdialyse, bietet die Peritonealdialyse verschiedene Vorteile, die von erhöhter Erwünschtheit sind. Zum Beispiel wurden automatische Vorrichtungen entwickelt, die es erlauben, daß der Patient sich einer Dialysebehandlung während des Schlafes in der Nacht unterziehen kann. Die Benutzung dieser automatischen Vorrichtung erlaubt dem Patienten größere Mobilität und Freiheiten tagsüber.

Die Peritonealdialyse kann auf mehrere Arten durchgeführt werden. Bei der CAPD (Continuous Ambulatory Peritoneal Dialysis) wird die Infusionslösung in die und aus der Peritonealhöhle geführt, wobei der Patient normale Handlungen den Tag über vollziehen kann. Der offensichtliche Nachteil der CAPD-Dialyse ist die lästige Vorrichtung , die der Patient benutzen muß. Beispiele von CAPD-Systemen werden in den amerikanischen Patentschriften Nr. 4,747,822 von Peabody und Nr. 4,620,846 von Goldberg gezeigt.

WO-A-84/03046 beschreibt eine Schlauchanordnung für eine Peritonealdialyse, deren Überführungsschlauch über ein Katheter-Verlängerungsstück mit dem implantierten Katheter verbunden ist. Die Verbindung zwischen dem Katheter-Verlängerungsstück und dem Überführungsschlauch kann durch einen Protektor geschütz sein.

Zwei Arten der Peritonealdialysetherapien, die besonders mit automatischen Systemen durchgeführt werden können, sind zum einen das IPD-System (Intermittent Peritoneal Dialysis) und das CCPD-System (Continuous Cycling Peritoneal Dialysis). Beim IPD-System zirkuliert zwischen mehreren Tage Behandlungsfreizeit eine große Menge Dialyselösung (bis zu 40 Litern) durch die Peritonealhöhle des Patienten über einen Zeitraum von 4 bis 24 Stunden. Beim CCPD-System erfolgt die Dialysebehandlung mehr oder weniger kontinuierlich mit einer Behandlungszeit von 3 bis 4 Stunden bei der Nacht, während der wache Patient tagsüber eine einzelne Charge Dialyselösung in der Peritonealhöhle aufweist. Es gibt dabei bestimmte Vorteile zu jedem dieser unterschiedlichen Therapietechniken.

Bei beiden Verfahren, sowohl bei der IPD als auch bei der CCPD arbeitet ein automatisches Dialysegerät in ähnlicher Art und Weise. Die Dialyselösung und die Schlauchanordnung sind eingegliedert mit einer Ventilanordnung, der Heizung und den Kontrollfunktionen, die mit der automatischen Apparatur verbunden sind. Bei vielen Systemen werden bestimmte Mengen Dialyselösung entweder gepumpt oder durch die Schwerkraft zugeführt. In der Heizstation wird die Lösung auf Körpertemperatur erwärmt, um einer unangenehmen Empfindung vorzubeugen, die beim Eintritt von raumwarmer oder kühlerer Flüssigkeit in die Peritonealhöhle auftritt. Die erwärmte Lösung wird dann von der Dialysestation zu der Peritonealhöhle des Patienten über einen Überführungsschlauch zugeführt, der im wesentlichen aus einem flexiblen Schlauch besteht, und in Verbindung steht mit dem Ende eines Katheters, der sich in der Peritonealhöhle des Patienten befindet. Nach einer Zeitperiode (Verweildauer) strömt die Lösung vom Patienten in einen Auffangbehälter ab.

Beim IPD-System zirkuliert eine große Menge Lösung über einen relativ kurzen Zeitraum. Wenn die Behandlung dann durchgeführt ist, ist der Patient wenigstens für einige Tage wieder unbelastet. Nachteilig ist es, daß eine sehr große Menge Dialyselösung benutzt werden muß. Behälter mit Inhalten von 40 Litern Lösung sind schwierig für einen Patienten in einem derartigen Krankheitsstadium zu heben. Beim CCPD- und CAPD-Verfahren erhält man die gleichen Ergebnisse durch die erhöhte Verweilzeit der Dialyselösung in der Peritonealhöhle. Die Gesamtmenge der erforderlichen Lösung kann deshalb signifikant vermindert werden. Der offensichtliche Nachteil ist, daß es weniger Erholungszeitraum von der Behandlung für den Patienten gibt.

Der Überführungsschlauch bei der PD-Therapie weist im allgemeinen einen flexiblen Schlauch zur Flüssigkeitsübertragung zu und von der Katheterseite, einen Katheter zur Einführung in die Peritonealhöhle und einen Konnektor zwischen dem Schlauch und dem Katheter auf. Eine freigebende betätigbare Klemme ist auf dem Schlauch angeordnet, um den Schlauch zu verschließen, wenn er mit dem Katheter durch den Konnektor verbunden ist. Weiterhin ist eine betätigbare Permanentklemme angeordnet, mit der der Schlauch am Ende der Dialyse permanent verschlossen werden kann, so daß der Schlauch von dem Konnektor getrennt werden kann, ohne daß die im Peritonealraum vorliegende Lösung ausläuft. Eine große Zahl von Varianten des PD-Schlauchsystems ist bekannt und wird auch in einer unterschiedlichen Vielzahl von Anwendungen verwendet.

Am Ende der PD ist es notwendig, den Patienten von der Schlauchanordnung zu trennen. Dabei wird in herkömmlicher Weise durch Schließen der ständig angeordneten Handklemme der Überführungsschlauch andauernd unterbrochen, wobei der Schlauch an der Seite der ständigen Handklemme auf der abgekehrten Seite vom Patienten durchgeschnitten wird. Dabei verschließt die ständig angeordnete Handklemme den Überführungsschlauch auf der Seite des Patienten, um ein Auslaufen der Flüssigkeit zu verhindern. Nachdem der Schlauch abgeschnitten ist, wird eine Klemme auf der Katheterseite dazu benutzt, den Katheter zu schließen. Beim nächsten PD-Sytemwechsel wird der Stummel der Schlauchleitung von dem Katheter abgezogen. Danach wird ein neues PD-System an den Katheter angeschlossen, wobei die Katheterklemme wieder geöffnet wird und der Prozeß sich wiederholt.

Das Zerschneiden des Überführungsschlauches wird üblicherweise mit einer Schere oder einem Messer duchgeführt. Die PD-Therapie zeichnet sich zweckmäßigerweise dadurch aus, daß sie durch den Patienten selbst durchgeführt werden kann, ohne die Hilfe einer Schwester oder einer anderen Person zu beanspruchen, wobei die Schere oder das Messer immer derart vorhanden sein muß, daß der Patient sie erreichen kann, während er noch mit der Schlauchanordnung verbunden ist.

Ein weiterer Nachteil ist, daß es auf dem Überführungsschlauch, der mit einem Messer oder einer Schere durchgeschnitten wird, keine Marke gibt, die anzeigt, wo geschnitten werden soll. Ein häufiges Problem dabei ist es, daß der Patient unglücklicherweise den Überführungsschlauch auf der Patientenseite der Klammer durchtrennt, so daß verbrauchte Flüssigkeit ausläuft. Weitaus schlimmer ist es, wenn der Patient unglücklicherweise den Katheter zerschneidet, so daß der zerstörte Katheter entfernt und duch einen neuen Katheter ersetzt werden muß.

Es ist daher die Aufgabe der Erfindung die Probleme der vorstehenden Art dahingehend zu lösen, daß ein zufälliges gefährliches Zertrennen des Überführungsschlauches verhindert wird.

Diese Aufgabe wird erfindungsgemäß mit den Merkmalen des Patentanspruchs 1 gelöst.

Die Schlauchleitung des Überführungsschlauches nach der Erfindung schließt eine Schlauchkupplung am Schlauchende ein, welches an den Katheter in der Peritonealhöhle des Patienten geht. Gemäß einer ersten Ausführungsform teilt die Kupplung den Überführungsschlauch in ein zur Peritonealhöhle proximales Segment und in ein distales Segment, wobei die beiden Segmente durch die Kupplung verbunden werden. Die Kupplung ist ein zylindrisches Hohlelement, wobei das eine Ende in das offene Ende des distalen Schlauchsegmentes und das andere Ende in das offene Ende des proximalen Schlauchsegmentes eingeführt ist. Die Schlauchkupplung wird in der Schlauchleitung selbst durch Reibschluß oder durch Lösungsmittelverschweißung gehalten. Eine ringförmige Lippe um den Umfang der Schlauchkupplung an oder benachbart zum mittleren Bereich dient vorteilhafterweise als Anstoßstelle für die Schlauchenden. Die Schlauchkupplung selbst ist um den Umfang in der Nähe der ringförmigen Lippe gekerbt.

Wenn nun das PD-Verfahren beendet ist, und der Überführungsschlauch hinreichend abgeklemmt ist, wird die Schlauchkupplung leicht an ihrer Sollbruchstelle durch starkes Biegen gebrochen. Dadurch wird weder eine Schere oder ein Messer benötigt, so daß keine Gefahr mehr besteht, den Überführungsschlauch auf der falschen Seite der Klemme oder sogar den Katheter selbst unbeabsichtigt durchzuschneiden.

Nach einer weiteren vorteilhaften Weiterbildung der Erfindung wird ein Y-Verbindungsstück als Rohranschlußstück verwendet. Von dem Y-Verbinder geht das erste Schlauchstück zum Patienten, das zweite Schlauchstück zu einem Beutel mit frischer Spüllösung und das dritte Schlauchstück zu einem Abfluß. Dabei ist die Sollbruchstelle gemäß dieser zweiten Ausführungsform um den Außenumfang des Y-Astes gelegt, der mit dem ersten Schlauchstück verbunden ist.

Weitere Einzelheiten, Merkmale und Vorteile der Erfindung ergeben sich aus der nachfolgenden Beschreibung von Ausführungsbeispielen anhand der Zeichnungen. Es zeigen:
- Fig. 1: eine Teilansicht der Schlauchanordnung gemäß der Erfindung zur Verwendung an einer automatischen Peritonealdialyseapparatur,
- Fig.2: eine gesonderte Ansicht der erfindungsgemäßen Kupplung, teilweise geschnitten dargestellt mit dem Überführungsschlauch der Schlauchanordnung aus Fig. 1,
- Fig. 3: eine gesonderte Ansicht gemäß der Erfindung, dargestellt ohne die Schlauchanordnung und
- Fig. 4: eine teilweise geschnittene Seitenansicht des erfindungsgemäßen Y-förmigen Rohranschlußstückes mit teilweise weggeschnittenen Schläuchen.

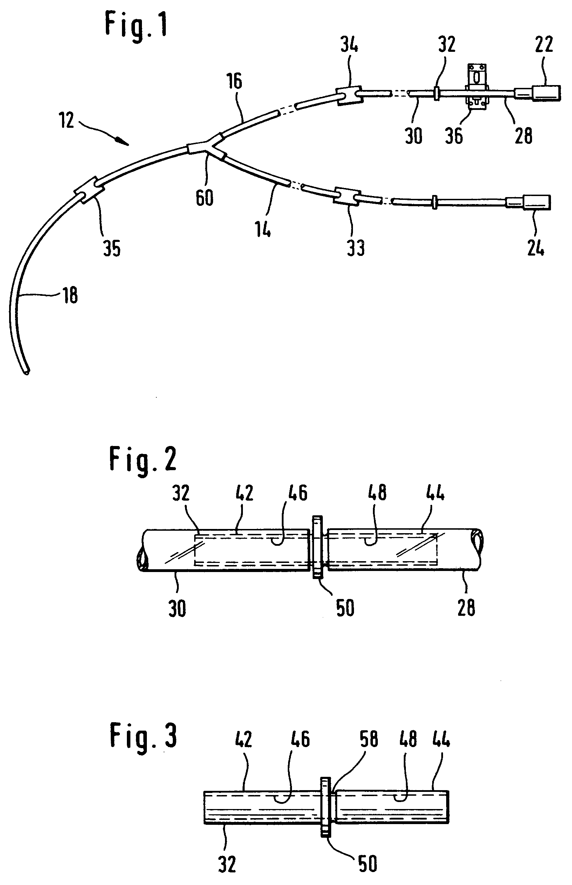

Fig. 1 zeigt einen Bereich 12 einer Schlauchanordnung zum Anschluß an eine automatische Peritonealdialyseapparatur, welche nicht dargestellt ist. Die Schlauchanordnung 12 schließt einen Zuführschlauch 14, einen Überführungsschlauch 16 zur Verbindung mit einen Peritonealkatheter, eine Ablaßleitung 18, die mit einem Abfallbehälter verbunden ist, und ein Y-förmiges Rohranschlußstück 60 zur Verbindung des Zuführungsschlauches 14, des Überführungsschlauches 16 und des Ablaßschlauches 18 ein. An dem Ende des Überführungsschlauches 16 ist ein Konnektor 22 zur Verbindung zu einem Peritonealkatheter angebracht. An dem Ende der Zuführleitung 14 ist ein weiterer Konnektor 24 zur Verbindung mit einer Anordnung nicht dargestellter Flüssigkeitsbehälter angeordnet.

Die Überführungsleitung 16 schließt einen Leitungsbereich 28, der der Peritonealhöhle des Patienten zugewandt ist, und einen Leitungsbereich 30, der zur Peritonealhöhle des Patienten abgewandt ist, und eine Schlauchkupplung 32 ein, die den abgewandten und zugewandten Bereich bezüglich der Peritonealhöhle in der nachbeschriebenen Weise verbindet. Weiterhin weist der Überführungsschlauch 16 eine lösbare Handklemme 34 an dem abgewandten Leitungsbereich 30 und eine permanente Handklemme 36 auf dem zugewandten Leitungsbereich 28 auf. Die Schlauchleitung selbst besteht aus einem herkömmlichen flexiblen, handelsüblichen Plastikschlauch für medizinische Zwecke. Die lösbare Handklemme 34 ist ein längliches vorgespanntes Glied mit offenen Enden und mit einem Klammerpaar an ihrer inneren Oberfläche, die auf den Schlauch klemmt, wenn das Glied zusammengedrückt wird. Eine Kante an einem der offenen Enden schnappt in eine Zahnanordnung auf dem anderen offenen Ende und hält so die Klemme auf der Schlauchleitung, wobei die Schlauchleitung teilweise oder vollständig abgeklemmt wird. Die Kante an einem der offenen Enden kann gelöst werden von der Zahnanordnung durch Wegbiegung des anderen offenen Endes von der Kante auf dem ersten offenen Ende. Klemmen diesen Typs sind an sich bekannt und werden hier nicht weiter beschrieben.

Die Permanentklemme 36 auf dem zugewandten Schlauchbereich 28 ist ebenfalls ein Klemmentyp, der nach seiner Art bekannt ist. Hierzu wäre kurz zu sagen, daß bei dieser Klemme zwei schwenkbare Hälften angeordnet sind, wobei die erste verschiebbar über den Schlauch greift und die zweite mit der ersten zusammenpaßt, wobei sie auf die erste durch ein Scharnier gefaltet wird. Die zweite Hälfte besitzt einen Vorsprung der den Schlauch zusammendrückt und hierdurch vollständig verschließt, wenn sie über die erste Hälfte gefaltet wird. Die erste Hälfte besitzt ein Hakenpaar, das in ein Paar von dazu passenden Schlitzen in der zweiten Hälfte eingreift, wodurch der Schlauch ständig und vollständig verschlossen wird.

Die Schlauchkupplung 32 ist besser zu erkennen in der Fig. 2, wo sie mit dem zugewandten Schlauchbereich 28 und dem abgewandten Schlauchbereich 30 verbunden ist und in Fig. 3 getrennt von den Schläuchen dargestellt ist. Die Schlauchkupplung 32 weist einen distalen Hohlzylinder 42 und eine proximalen Hohlzylinder 44 auf. Jeder dieser Zylinder ist ein längliches Teil mit einer axialen Bohrung 46 bzw. 48. Der Außendurchmesser des Hohlzylinders 42 und 44 verjüngt sich zu einem schmaleren Durchmesser zu jedem der Enden hin. Vorzugsweise sind die Außendurchmesser geringfügig größer als der Innendurchmesser des eingesetzten Schlauches, so daß der Hohlzylinder in die Schläuche gepreßt werden kann, um einen paßgenauen und wasserdichten Reibschluß zwischen der Schlauchkupplung 32 und den Schläuchen zu erreichen. Der Innendurchmesser der Hohlzylinder ist dabei so groß wie möglich, so daß der Fluß durch die Kupplung nicht übermäßig behindert wird, jedoch ist der Innendurchmesser nicht so groß, daß die Wanddicke des Hohlzylinders so dünn wird, daß die Schlauchkupplung 32 unter normalen Handhabungsbedingungen bricht. Es wurde herausgefunden, daß beim Einsatz von Plastikschläuchen aus PVC für medizinische Zwecke und einer Kupplung aus Polycarbonat diese Bedingungen erfüllt werden können, wenn ein Innendurchmesser von 4 mm, ein maximaler Außendurchmesser von 6,15 mm und ein minimaler Außendurchmesser an beiden Enden von 5,8 mm vorliegen.

Zwischen dem distalen Hohlzylinder 42 und dem proximalen Hohlzylinderteil 44 der Schlauchkupplung 32 ist ein erhöhtes ringförmiges Anstoßelement 50 angebracht, welches sich über den Umfang der Hohlzylinder 42 und 44 erstreckt. Das ringförmige Anstoßelement 50 bewirkt einen Anhalt für den abgewandten Schlauchbereich 30 und dem zugewandten Schlauchbereich 28, so daß jeder von ihnen mit einem entsprechenden Längenbetrag auf die Kupplung geschoben werden kann, wodurch eine sichere und wasserdichte Verbindung erhalten wird. Bei der bevorzugten Vorrichtung hat jeder der Hohlzylinder 42 und 44 eine Länge von über 25,4 mm und das ringförmige Anstoßelement einen Außendurchmesser von 37,9 mm.

Der proximale Hohlzylinder 44 der Schlauchkupplung 32 weist eine Sollbruchstelle bildende Kerbe 58 am Außenumfang in der Nachbarschaftslage zu dem erhöhten ringförmigen Anstoßelement 50 auf, so daß die Kupplung, wie unten beschrieben wird, gebrochen werden kann. Es hat sich herausgestellt, daß bei einer Dicke der Kupplungswand von 1mm eine Dicke für die Kerbtiefe von annähernd 0,63 mm als ausreichend anzusehen ist, damit die Kupplung leicht gebrochen werden kann, ohne daß die Kupplung so geschwächt wird, daß sie ungewollt bricht. Die Kerbe 58 kann auch auf der distalen Hohlzylinderseite 42 und nicht auf der proximalen Hohlzylinderseite 44 angebracht sein. In beiden Fällen ist die Kerbe 58 vorzugsweise angrenzend zu dem erhöhten, ringförmigen Anschlußelement 50 angebracht, so daß die Bruchstelle nicht nennenswert von einem der Schläuche überlappt wird, was ansonsten die Trennung der Kupplungsteile behindern würde.

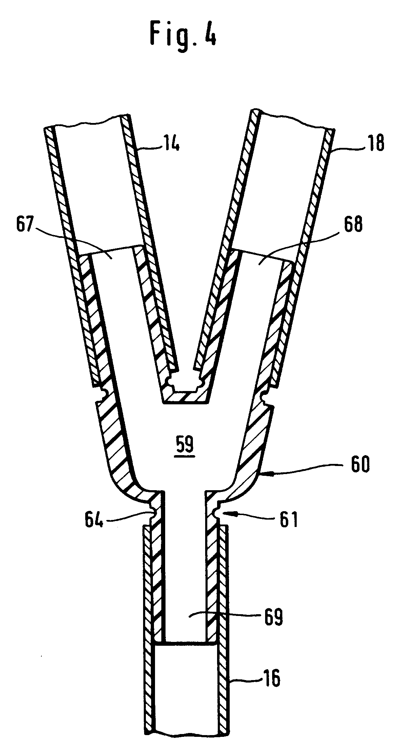

Nach einer weiteren vorteilhaften Ausführungsform der Erfindung, dargestellt in den Figuren 4 und 5, ist das Mittel zum Abtrennen im benachbarten Bereich zur Verzweigungstelle 59 des Zweiges 69 des Y-förmigen Rohranschlußstückes 60 gelegen, der noch zwei weitere Zweige 67 und 68 aufweist. Die Zweige 67-69 sind, wie aus Figur 4 ersichtlich, mit den Schlauchstücken 14, 18, 16 verbunden. Dabei ist das Mittel zum Abtrennen als Sollbruchstelle 61 ausgebildet. Die Sollbruchstelle 61 weist, wie die Schlauchkupplung 32, eine Kerbe 64 auf, die sich über den gesamten Umfang des Y-Zweiges 69 erstrecken kann. Aufgrund des Abtrennmittels am Zweig 69 des Y-förmigen Rohranschlußstückes 60 kann auf leichte und sichere Weise die abzutrennende Schlauchleitung 16 vom Y-förmigen Rohranschlußstückes 60 getrennt werden.

Wie bereits oben schon ausgeführt, sollte die Schlauchkupplung 32 und das Y-förmige Rohranschlußstück 60 aus Hartkunststoff, insbesondere Polycarbonat bestehen und als Spritzgrußformteil hergestellt sein. Andere Materialien und Fabrikationsmethoden sind für den Fachmann offensichtlich.

Zur Behandlung ist die Schlauchanordnung 12 derart angeordnet, daß der Zuführschlauch 14 mit einem oder mehreren nicht gezeigten Flüssigkeitsbehältern verbunden wird, die eine für die PD-taugliche Lösung enthalten. Ein Abfluß wird passend auf eine Position gestellt, die tiefer liegt als der Peritonealraum des Patienten. Der Überführungsschlauch 16 wird mit dem Katheter verbunden, der in die Peritonealhöhle des Patienten reicht. Die freigebenden Handklemmen 34 und 35 auf dem Überführungsschlauch 16 und auf dem Abflußschlauch 16 sind geöffnet, so daß die gebrauchte Flüssigkeit üblicherweise durch Schwerkraftförderung zum Abfluß gelangt. Anschließend wird die Klemme 35 geschlossen und es wird die Klemme 33 auf den Überführungsschlauch 16 geöffnet, so daß frische PD-Lösung von dem nicht gezeigten, an dem Konnektor 56 angeschlossenen Behälter durch die Schlauchstücke 14 und 16 in den Peritonealraum strömen kann. Danach werden die Klemme 34 und die Permanenthandklemme 36 geschlossen, um den Überführungsschlauch 16 gänzlich zu schließen. Der Überführungsschlauch 16 wird dann zwischen dem zugewandten Teil 28 und dem abgewandten Teil 30 durch Brechen der Schlauchkupplung 32 gemäß der ersten Ausführungsform getrennt. Der Bruchvorgang der Schlauchkupplung 32 wird dabei derart vollzogen, daß jedes Ende erfaßt wird und die Kupplung selbst derart gebogen wird, daß sie sauber entlang der Kerbe 58 bricht. Der zugewandte Schlauchbereich 28 kann dann bei einem Systemwechsel von dem Katheter entfernt werden.

Gemäß einer weiteren Ausführungsform wird das Y-Stück 60 einerseits an den beiden Ästen 67 und 68 sowie andererseits an dem Ast 69 erfaßt und so verbogen, daß die Sollbruchstelle 64 abbricht und somit den Ast 69 freigibt. Aufgrund dieses Trennungsvorganges des Überführungsschlauches 16 durch Brechen der Schlauchkupplung 32 oder des Y-Stückes 60 wurde erreicht, daß weder ein Messer noch eine Schere oder andere Werkzeuge zur Durchführung des Trennvorganges benutzt werden müssen.

Darüber hinaus sichert die Schlauchanordnung 12 zur Trennung des Überführungsschlauches 16, daß die Trennung ausschließlich an der Schlauchkupplung 32 oder dem Y-Stück 60 stattfindet und nicht auf der falschen Seite der Klemme oder an einer anderen unkorrekten Stelle, zumal die Permanentklemme 36 werkseits in die Position zwischen Sollbruchstelle 58 bzw. 64 und patienten-seitigem Konnektor 22 gebracht worden ist. Es kann abschließend gesagt werden, daß das Ergebnis ein einfaches, billiges, sicheres und zuverlässiges System ist. Obgleich die Schlauchkupplung 32 bzw. der Y-Konnektor 60 in Verbindung mit einer Peritonealdialyse beschrieben wird, ist es augenscheinlich, daß die Schlauchkupplung 32 ebenfalls Verwendung für andere Dialysetypen und für andere medizinische und nicht medizinische Anwendungen findet.

## Patentansprüche

1. Schlauchanordnung für eine Peritonealdialyse zur Verabreichung einer Spülflüssigkeit in die Peritonealhöhle eines Patienten durch einen Peritonealkatheter und zum Abführen der verbrauchten Spülflüssigkeit aus der Peritonealhöhle in einen Abfluß mit
einem Zuführungsschlauch (14), der mit einem die frische Spülflüssigkeit aufweisenden Behälter in Strömungsverbindung steht,
einem Überführungsschlauch (16), der einerseits mit dem Peritonealkatheter und andererseits mit dem Zuführungsschlauch (14) in Strömungsverbindung steht,
einem Abführungsschlauch (18), der einerseits mit dem Überführungsschlauch (16) und andererseits mit dem Abfluß in Strömungsverbindung steht,
einem Rohranschlußstück (60), an dessen Rohrschenkeln der Zuführungsschlauch (14), der Überführungsschlauch (16) und der Abführungsschlauch (18) angeschlossen sind,
Klemmen (33-35), mit denen diese Schläuche (14, 16, 18) absperrbar sind, und
Mitteln zum Abtrennen des Überführungsschlauchs (16) von dem Zuführungsschlauch (14) und dem Abführungsschlauch (18),
dadurch gekennzeichnet,
daß die Mittel zum Abtrennen des Überführungsschlauchs (16) auf dem mit dem Überführungsshlauch (16) verbundenen Rohrschenkel (69) des Rohranschlußstücks (60) vorgesehen sind oder daß die Mittel zum Abtrennen des Überführungsschlauchs durch eine in den Überführungsschlauch eingefügte mit einer Sollbruchstelle (61) versehene rohrförmige Schlauchkupplung (32) gebildet werden, die ein distales Ende, welches mit dem abgewandten Schlauchbereich (30) des Überführungsschlauchs (16) verbunden ist und ein proximales Ende aufweist, welches mit dem zugewandten Schlauchbereich (28) des Überführungsschlauchs (16) verbunden ist, wobei das distale und das proximale Ende voneinander trennbar sind.

2. Schlauchanordnung nach Anspruch 1, dadurch gekennzeichnet, daß das distale Ende der Schlauchkupplung (32) als ein Hohlzylinder in Strömungsverbindung mit dem Überführungsschlauch (16) des abgewandten Schlauchbereichs (30) steht und das proximale Ende der Schlauchkupplung (32) ebenfalls ein Hohlzylinder ist, der in Strömungsverbindung mit dem Überführungsschlauch (16) des zugewandten Schlauchbereichs (28) in Verbindung steht, wobei der Hohlzylinder (42, 44) einen Außendurchmesser hat, der größer ist als der Innendurchmesser des Schlauchs, mit dem sie in Strömungsverbindung verbunden sind, und der Hohlzylinder (42, 44) in den Hohlraum des Schlauches einführbar ist, um eine wasserdichte Verbindung herzustellen.

3. Schlauchanordnung nach Anspruch 1 oder 2, dadurch gekennzeichnet, daß an der Außenoberfläche der Schlauchkupplung (32) oder an dem mit dem Überführungsschlauch (16) verbundenen Rohrschenkel (69) des Rohranschlußstücks (60) eine Kerbe (58, 64) vorgesehen ist, wobei die Schlauchkupplung (32) oder der Rohrschenkel (69) entlang der Kerbe (58, 64) durch eine Biegekraft brechbar ist.

4. Schlauchanordnung nach einem der Ansprüche 1 bis 3, dadurch gekennzeichnet, daß die Schlauchkupplung (32) ein Anstoßelement (50) aufweist, das zur Festlegung der Einführung des Hohlzylinders (42, 44) in die Schlauchöffnung bis zu einer bestimmten Einführlänge dient.

## Claims

1. A peritoneal dialysis tubing administration set for use in administering flushing fluid into a patient's peritoneal cavity through a peritoneal catheter and for discharging the spent fluid from the peritoneal cavity into a drain channel comprising
a fluid supply tube (14) in flow communication with the fresh flushing fluid container,
a fluid delivery tube (16) which on the one end is in flow communication with the peritoneal catheter and on the other hand with the fluid supply tube (14),
a waste discharge tube (18) which is in flow communication on the one hand with the fluid delivery tube (16) and on the other hand with the drain channel,
a tube connector piece (60) of which the branches are connected to the fluid supply tube (14), the fluid delivery tube (16) and the drain channel (18),
clamps (33-35) which can be used to occlude these tubes (14, 16, 18), and
means for separatingthe fluid delivery tube (16) from the fluid supply tube (14) and the drain channel (18),
characterized in
that said means for separating the fluid delivery tube (16) are provided on said tube branch (69) of said tube connector piece (60) connected to said fluid delivery tube (16) or that said means for separating the fluid delivery tube are formed by a tubular tubing coupling (32) inserted into said fluid delivery tube and provided with a predetermined breaking point (61) and which comprise a distal end being connected to the distal tubing section (30) of the fluid delivery tube (16) and a proximal end being connected to the proximal tubing section (28) of said fluid delivery tube (16) with the distal and the proximal end being able to be separated from one another.

2. The tubing administration set according to claim 1, characterized in that said distal end of said tubing coupling (32) is a hollow cylinger in flow communication with said fluid delivery tube (16) of the distal tube section (30) and thatsaid proximal end of said tubing coupling (32), too, is a hollow cylinder in flow communication with said fluid delivery tube (16) of the proximal tube section, wherein the hollow cylinders (42, 44) have an outer diameter being greater than the inner diameter of the tube with which they are in flow communication, and that the hollow cylinder (42, 44) may be inserted into the lumen of the tube for establishing a water-tight connection.

3. The tubing administration set according to claim 1 or 2, characterized in that at the outer surface of said tubing coupling (32) or at the tube branch (69) of said connector piece (60) connected to the fluid supply tube (16) a scoring (58, 64) is provided, with the tubing coupling (32) or the tube branch (69) being breakable along the scoring (58, 69) by a bending force.

4. The tubing administration set according to any of claims 1 to 3, characterized in that said tubing coupling (32) includes a stop means (50) for halting the insertion of said hollow cylinders (42, 44) into the tubing aperture at a predetermined insertion length.

## Revendications

1. Jeu de flexibles pour une dialyse péritonéale servant à l'introduction d'un liquide d'épuration dans la cavité péritonéale d'un patient par l'intermédiaire d'un cathéter péritonéal et à l'évacuation du liquide d'épuration usé hors de la cavité péritonéale dans un flux d'évacuation comprenant
un flexible d'amenée (14), qui est raccordé en communication d'écoulement avec un récipient contenant le liquide d'épuration frais,
un flexible de transfert (16), qui est raccordé en communication d'écoulement, d'une part, au cathéter péritonéal et, d'autre part, au flexible d'amenée (14),
un flexible d'évacuation (18), qui est raccordé en communication d'écoulement, d'une part, au flexible de transfert (16) et, d'autre part, à l'évacuation,
d'un raccord (60), aux tubulures duquel sont raccordés le flexible d'amenée (14), le flexible de transfert (16) et le flexible d'évacuation (18),
des pinces (33-35), au moyen desquelles ces flexibles (14, 16, 18) peuvent être fermés, et
des moyens pour séparer le flexible de transfert (16) du flexible d'amenée (14) et du flexible d'évacuation (18),
caractérisé en ce que les moyens pour séparer le flexible de transfert (16) sont prévus sur la tubulure (69) du raccord (60) raccordée au flexible de transfert (16) ou que les moyens pour séparer le flexible de transfert sont formés par un raccord flexible tubulaire (32), pourvu d'un endroit destiné à la rupture (61) et inséré dans le flexible de transfert, qui présente une extrémité distale, qui est raccordée à la région (30), éloignée du patient, du flexible de transfert (16), et une extrémité proximale, qui est raccordée à la région (28), proche du patient, du flexible de transfert (16), étant entendu que l'extrémité distale et l'extrémité proximale peuvent être séparées l'une de l'autre.

2. Jeu de flexibles suivant la revendication 1, caractérisé en ce que l'extrémité distale du raccord flexible (32) est raccordée en communication d'écoulement en tant que cylindre creux au flexible de transfert (16) de la région de flexible (30) éloignée de l'entrée et l'extrémité proximale du raccord flexible (32) est également un cylindre creux, qui est raccordé en, communication d'écoulement avec le flexible de transfert (16) de la région de flexible (28) proche du patient, étant entendu que le cylindre creux (42, 44) a un diamètre extérieur, qui est supérieur au diamètre intérieur du flexible, auquel il est raccordé en communication d'écoulement, et le cylindre creux (42, 44) peut être inséré dans l'espace creux du flexible, afin d'établir un raccordement étanche à l'eau.

3. Jeu de flexibles suivant la revendication 1 ou 2, caractérisé en ce que dans la surface extérieure du raccord flexible (32) ou dans la tubulure (69) du raccord (60) raccordée au flexible de transfert (16) est prévue une entaille (58, 64), le raccord flexible (32) ou la tubulure (69) pouvant être rompu suivant l'entaille (58, 64) par un effort de flexion.

4. Jeu de flexibles suivant l'une quelconque des revendications 1 à 3, caractérisé en ce que le raccord flexible (32) présente un organe de butée (50), qui sert à arrêter l'introduction du cylindre creux (42, 44) dans l'orifice du flexible à une longueur d'introduction imposée.
